# EUROPEAN PATENT APPLICATION

(11) **EP 4 487 770 A1**
(43) Date of publication of application: **08.01.2025**
(21) Application number: 22929750.2
(22) Date of filing: 02.03.2022
(51) Int. Cl.: A61B 5/1171

(54) **INFORMATION PROCESSING DEVICE, INFORMATION PROCESSING METHOD, AND RECORDING MEDIUM**

(71) Applicant: NEC Corporation, 108-8001 Tokyo (JP)
(72) Inventor: KAJIKI, Yoshihiro, Tokyo 108-8001 (JP)
(74) Representative: Betten & Resch
(86) International application number: PCT/JP2022/008837
(87) International publication number: WO 2023/166603

(57) **Abstract**

An information processing apparatus 3 includes: an applying unit 311 that applies a stimulus including at least one of light, a picture, and a sound that induces a change in a vital reaction of a target person; and a determination unit 312 that determines whether or not the target person is a living body, on the basis of a correlation between the stimulus and the vital reaction that occurs in the target person to which the stimulus is applied.

## Description

### Technical Field

This disclosure relates to, for example, technical fields of an information processing apparatus, an information processing method, and a recording medium that are configured to determine whether a target person is a living body.

### Background Art

Patent Literature 1 describes an example of an information processing apparatus that is configured to determine whether or not a target person is a living body.

### Citation List

### Patent Literature

Patent Literature 1: JP2000-033080A

### Summary

### Technical Problem

It is an example object of this disclosure to provide an information processing apparatus, an information processing method, and a recording medium that are intended to improve the techniques/technologies described in Citation List.

### Solution to Problem

An information processing apparatus according to an example aspect of this disclosure includes: an applying unit that applies a stimulus including at least one of light, a picture, and a sound that induces a change in a vital reaction of a target person; and a determination unit that determines whether or not the target person is a living body, on the basis of a correlation between the stimulus and the vital reaction that occurs in the target person to which the stimulus is applied.

An information processing method according to an example aspect of this disclosure includes: applying a stimulus including at least one of light, a picture, and a sound that induces a change in a vital reaction of a target person; and determining whether or not the target person is a living body, on the basis of a correlation between the stimulus and the vital reaction that occurs in the target person to which the stimulus is applied.

A recording medium according to an example aspect of this disclosure is a recording medium on which a computer program that allows a computer to execute an information processing method is recorded, the information processing method including: applying a stimulus including at least one of light, a picture, and a sound that induces a change in a vital reaction of a target person; and determining whether or not the target person is a living body, on the basis of a correlation between the stimulus and the vital reaction that occurs in the target person to which the stimulus is applied.

### Brief Description of Drawings

[FIG. 1] FIG. 1 is a block diagram illustrating a configuration of an information processing apparatus in a first example embodiment.
[FIG. 2] FIG. 2 is a block diagram illustrating an overall configuration of an authentication system in a second example embodiment.
[FIG. 3] FIG. 3 is a block diagram illustrating a configuration of a spoofing determination apparatus in the second example embodiment.
[FIG. 4] FIG. 4 is a flowchart illustrating a flow of a spoofing determination operation performed by the spoofing determination apparatus in the second example embodiment.
[FIG. 5] FIG. 5 is a timing chart illustrating a diameter of a pupil calculated on the basis of a person image in which an authentication subject who is a living body is captured as an imaged person, when a stimulus including light irradiated toward an eye of a living body, is applied to the living body, and a diameter of a pupil calculated on the basis of a person image in which an imitation that is a non-living body is captured as the imaged person.
[FIG. 6] FIG. 6 is a plan view illustrating feature points (in other words, landmarks) of the imaged person.
[FIG. 7] FIG. 7 is a timing chart illustrating an example of a correlation between an eye size and the stimulus.
[FIG. 8] FIG. 8 is a plan view illustrating an example of a picture that indirectly induces adjustment of focus of a human crystalline lens.
[FIG. 9] FIG. 9 is a plan view illustrating an example of a picture that indirectly induces the adjustment of the focus of the human crystalline lens.
[FIG. 10] FIG. 10A is a cross-sectional view illustrating reflected light emitted from a relatively thin crystalline lens, and FIG. 10B is a cross-sectional view illustrating reflected light emitted from a relatively thick crystalline lens.
[FIG. 11] FIG. 11 is a timing chart illustrating an example of a correlation between each of a direction in which a face is directed and a direction of a line of sight is directed, and the stimulus.

### Description of Example Embodiments

Hereinafter, with reference to the drawings, an information processing apparatus, an information processing method, and a recording medium according to example embodiments will be described.

### (1) First Example Embodiment

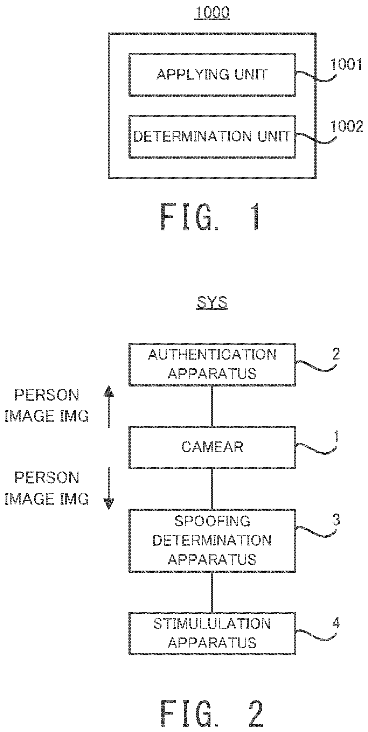

First, an information processing apparatus, an information processing method, and a recording medium in a first example embodiment will be described. With reference to FIG. 1, the following describes the information processing apparatus, the information processing method, and the recording medium in the first example embodiment, by using an information processing apparatus 1000 to which the information processing apparatus, the information processing method, and the recording medium in the first example embodiment are applied. FIG. 1 is a block diagram illustrating a configuration of the information processing apparatus 1000 in the first example embodiment.

The information processing apparatus 1000 is configured to determine whether or not a target person is a living body. In order to determine whether or not the target person is a living body, as illustrated in FIG. 1, the information processing apparatus 1000 includes an applying unit 1001 and a determination unit 1002. The applying unit 1001 applies a stimulus that induces a vital reaction of the target person. The stimulus that induces the vital reaction of the target person may include at least one of a light, a picture, and a sound. The determination unit 1002 determines whether or not the target person is a living body on the basis of a correlation between the stimulus and the vital reaction of the stimulated target person.

The information processing apparatus 1000 in the first example embodiment is capable of properly determining whether or not the target person is a living body, as compared with an information processing apparatus in a first comparative example that determines whether or not the target person is a living body without using the correlation between the stimulus and the vital reaction of the stimulated target person. Details of the reason will be described in a second example embodiment.

### (2) Second Example Embodiment

Next, an information processing apparatus, an information processing method, and a recording medium in a second example embodiment will be described. The following describes the information processing apparatus, the information processing method, and the recording medium in the second example embodiment, by using an authentication system SYS to which the information processing apparatus, the information processing method, and the recording medium in the second example embodiment are applied.

The authentication system SYS uses a person image IMG generated by a camera 1 imaging a person present in front of the camera 1 (hereafter referred to as an "authentication subject") (see FIG. 2 described later), thereby authenticating the authentication subject. Specifically, the authentication system SYS may authenticate the authentication subject by performing verification processing for determining whether or not a person captured in the person image IMG matches a person previously registered in the authentication system SYS (hereinafter referred to as a "registered person").

The authentication system SYS may acquire biometric information about the authentication subject from the person image IMG, and may perform biometric authentication for authenticating the authentication subject on the basis of the acquired biometric information. For example, when a face of the authentication subject is captured in the person image IMG, the authentication system SYS may perform face authentication for authenticating the authentication subject on the basis of the face of the authentication subject. When an iris of the authentication subject is captured in the person image IMG, the authentication system SYS may perform iris authentication for authenticating the authentication subject on the basis of a pattern of the iris of the authentication subject. When a fingerprint of the authentication subject is captured in the person image IMG, the authentication system SYS may perform fingerprint authentication for authenticating the authentication subject on the basis of a pattern of the fingerprint of the authentication subject. When a vein of the authentication subject is captured in the person image IMG, the authentication system SYS may perform vein authentication for authenticating the authentication subject on the basis of a pattern of the vein of the authentication subject.

Here, the camera 1 usually images a person actually present in front of the camera 1. In this situation, a person captured in the person image IMG (hereinafter referred to as an "imaged person") matches the authentication subject actually present in front of the camera 1. Therefore, the verification processing for determining whether or not the imaged person captured in the person image IMG matches the registered person, is equivalent to verification processing for determining whether or not the authentication subject present in front of the camera 1 matches the registered person. Therefore, when it is determined that the imaged person matches the registered person, the authentication subject matches the registered person, and thus, the authentication system SYS determines that the authentication of the authentication subject (i.e., the authentication of the imaged person captured in the person image IMG, and the same shall apply hereinafter) is successful. On the other hand, when it is determined that the imaged person does not match the registered person, the authentication subject does not match the registered person, and thus, the authentication system SYS determines that the authentication of the authentication person is failed.

On the other hand, a malicious authentication subject is likely to make the camera 1 image an imitation that imitates the registered person who is different from the authentication subject, so as to impersonate/pretend the registered person who is different from the authentication subject. An example of the imitation is a photograph in which the registered person is captured. An example of the imitation is a display that displays an image of the registered person. An example of the imitation is a mask that imitates a face of the registered person. An example of the imitation is an object that imitates a vein pattern or a finger pattern of the registered person. An example of the imitation is a robot that imitates the registered person. In this situation, the camera 1 images not the authentication subject actually present in front of the camera 1, but the imitation. In this case, even though there is no registered person actually present in front of the camera 1, the registered person is captured as the imaged person (actually, the imitation that imitates the registered person is captured as the imaged person) in the person image IMG generated by the camera 1. Consequently, the authentication systematic SYS is likely to determine that the imaged person captured in the person image IMG (in this instance, the person imitated by the imitation) matches the registered person by performing the verification processing. Therefore, the authentication system SYS may erroneously determine that the authentication of the authentication subject is successful. Specifically, the authentication system SYS may erroneously determine that the authentication subject actually present in front of the camera 1 matches the registered person, even though the registered person is not actually in front of the camera. In other words, the authentication system SYS is likely to erroneously determine that the authentication of the authentication subject is successful, even though it should be determined that the authentication of the authentication subject is failed, because the authentication subject who is different from the registered person is in front of the camera 1.

Therefore, the authentication system SYS that authenticates the authentication subject is required to prevent the malicious authentication subject from impersonating the registered person who is different from the authentication subject. Thus, the authentication system SYS performs a spoofing determination operation of determining whether or not the authentication subject impersonates another person, in addition to an authentication operation of authenticating the authentication subject. Specifically, the authentication system SYS performs the spoofing determination operation of determining whether or not the authentication subject present in front of the camera 1 impersonates another person, by determining whether or not the imaged person captured in the person image IMG is a living body (specifically, whether or not it is an imitation).

Hereinafter, details of a configuration and operation of the authentication system SYS that performs such a spoofing determination operation will be described in order.

### (2-1) Overall Configuration of Authentication System SYS in Second Example Embodiment

First, an overall configuration of the authentication system SYS in the second example embodiment will be described with reference to FIG. 2. FIG. 2 is a block diagram illustrating the overall configuration of the authentication system SYS in the second example embodiment.

As illustrated in FIG. 2, the authentication system SYS includes a camera 1, an authentication apparatus 2, a spoofing determination apparatus 3 that may be referred to as an information processing apparatus, and a stimulation apparatus 4.

The camera 1 is configured to image an imaging target range. Typically, in the imaging target range, there is the authentication subject actually present in front of the camera 1. In this situation, the camera 1 images the authentication subject, thereby generating the person image IMG in which the authentication subject is captured as the imaged person. Alternatively, as described above, when the authentication subject impersonates the registered person by using the imitation that imitates the registered person, there is the imitation in the imaging target range. In this case, the camera 1 images the imitation, thereby generating the person image IMG in which the registered person who is different from the authentication subject, is captured as the imaged person. The camera 1 outputs the generated person image IMG to the authentication apparatus 2 and the spoofing determination apparatus 3.

The authentication apparatus 2 acquires the person image IMG from the camera 1. The authentication apparatus 2 performs an authentication operation of authenticating the authentication subject by using the acquired person image IMG. That is, the authentication apparatus 2 determines whether or not the imaged person captured in the person image IMG is the same as the registered person, by using the acquired person image IMG. When it is determined that the imaged person is the same as the registered person, it is determined that the authentication of the authentication subject is successful. On the other hand, when it is determined that the imaged person is not the same as the registered person, it is determined that the authentication of the authentication subject is failed.

The authentication apparatus 2 may acquire the biometric information about the imaged person captured in the person image IMG from the person image IMG, and may perform the biometric authentication for authenticating the authentication subject on the basis of the acquired biometric information. In this instance, the above-described imitation may be regarded as an object obtained by duplicating the biometric information about the registered person. An example of the biometric information includes at least one of information about the face, information about the pattern of the iris, information about the pattern of the fingerprint, and information about the pattern of the vein, as described above.

The spoofing determination apparatus 3 acquires the person image IMG from the camera 1. The spoofing determination apparatus 3 performs a spoofing determination operation of determining whether of not the authentication subject impersonates another person, by using the acquired person image IMG. Specifically, the spoofing determination apparatus 3 detects the vital reaction of the imaged person captured in the person image IMG on the basis of the acquired person image IMG. In particular, the spoofing determination apparatus 3 detects the vital reaction of the imaged person captured in the person image IMG, while applying to the imaged person a stimulus that induces a change in the vital reaction of the imaged person captured in the person image IMG. Thereafter, the spoofing determination apparatus 3 determines whether or not the authentication subject impersonates another person on the basis of the detected vital reaction.

When the camera 1 images the authentication subject actually present in front of the camera 1, the stimulus to be applied to the imaged person in order to induce a change in the vital reaction of the imaged person captured in the person image IMG, is applied to the authentication subject who is the same as the imaged person. In this instance, due to the stimulus applied to the authentication subject, the vital reaction of the authentication subject should change in an aspect of correlating with the applied stimulus. Therefore, the camera 1 images the authentication subject whose vital reaction changes in the aspect of correlating with the applied stimulus. As a consequence, the vital reaction of the imaged person captured in the person image IMG, also changes in the aspect of correlating with the applied stimulus. Therefore, it is assumed that, when the vital reaction of the imaged person captured in the person image IMG changes in the aspect of correlating with the applied stimulus, the imaged person captured in the person image IMG is the same as the stimulated authentication subject. That is, the imaged person captured in the person image IMG is assumed to be a living body that is not the imitation. Consequently, it is assumed that the authentication subject does not impersonate another person.

On the other hand, when the camera 1 images the imitation that imitates the registered person, the stimulus to be applied to the imaged person in order to induce a change in the vital reaction of the imaged person captured in the person image IMG, is actually applied to the authentication subject who is different from the imaged person. Even in this case, due to the stimulus applied to the authentication subject, the vital reaction of the authentication subject changes in the aspect of correlating with the applied stimulus. The camera 1, however, images not the authentication subject whose vital reaction changes in the aspect of correlating with the applied stimulus, but the imitation in which the vital reaction does not change. Consequently, it is unlikely that the vital reaction of the imaged person captured in the person image IMG changes in the aspect of correlating with the applied stimulus. Therefore, it is assumed that, when the vital reaction of the imaged person captured in the person image IMG does not change in the aspect of correlating with the applied stimulus, the imaged person captured in the person image IMG is not the same as the stimulated authentication subject. That is, it is assumed that the imaged person captured in the person image IMG is the imitation that is not a living body. Consequently, it is assumed that the authentication subject impersonates another person.

As described above, in the second example embodiment, the spoofing determination apparatus 3 applies the stimulus that induces the vital reaction of the imaged person captured in the person image IMG, to the imaged person (actually, the authentication subject, and the same shall apply hereinafter), and determines whether or not the imaged person captured in the person image IMG is a living body on the basis of the correlations between the vital reaction of the imaged person captured in the person image IMG and the applied stimulus. When it is determined that the imager person captured in the person image IMG is a living body, the spoofing determination apparatus 3 determines that the authentication subject does not impersonate another person. When it is determined that the imaged person captured in the person image IMG is not a living body, the spoofing determination apparatus 3 determines that the authentication subject impersonates another person.

The spoofing determination apparatus 3 applies the stimulus to the imaged person by using the stimulation apparatus 4. Therefore, the stimulation apparatus 4 includes an apparatus that is configured to generate the stimulus to be applied to the imaged person.

For example, the vital reaction of a living body may change due to light irradiated/illuminated to the living body. Therefore, the stimulation apparatus 4 may apply to the imaged person the stimulus that induces the vital reaction of the imaged person, by irradiating the imaged person with light. In this instance, the stimulation apparatus 4 may include a light irradiation apparatus that is configured to irradiate the imaged person with light.

For example, the vital reaction of a living body may change due to a picture displayed toward the living body. Therefore, the stimulation apparatus 4 may apply to the imaged person the stimulus that induces the vital reaction of the imaged person, by displaying (i.e., outputting) a picture toward the imaged person. In this instance, the stimulation apparatus 4 may include a display apparatus (i.e., a display) that is configured to display a picture toward the imaged person. The "picture" in the second example embodiment may mean at least one of a video and a still image.

For example, the vital reaction of a living body may change due to a sound outputted toward the living body. Therefore, the stimulation apparatus 4 may apply to the imaged person the stimulus that induces the vital reaction of the imaged person, by outputting a sound toward the imaged person. In this instance, the stimulation apparatus 4 may include a sound output apparatus (i.e., a speaker) that is configured to output a sound toward the imaged person.

The authentication system SYS may be used to manage entry and exit of the authentication subject to a restricted area, for example. Specifically, the restricted area is an area where the authentication subject who meets a predetermined entry condition is permitted to enter, while the authentication subject who does not meet the prescribed entry condition is not permitted (i.e., prohibited) to enter. In this situation, the authentication apparatus 2 may authenticate the authentication subject by determining whether or not the authentication subject is the same as the registered person corresponding to a person who is permitted to enter the restricted area.

The authentication system SYS may be used to perform identity verification of the authentication subject who makes a commercial transaction, for example. In such a case, the authentication apparatus 2 may authenticate the authentication subject by determining whether or not the authentication subject who is about to make a commercial transaction with a transactee/customer, is the same as the registered person corresponding to a person who is recognized as a trading partner by the transactee/customer.

The authentication system SYS may be implemented as a single apparatus that is configured to function as the authentication apparatus 2 and the spoofing determination apparatus 3, as well as including the camera 1 and the stimulation apparatus 4. For example, the authentication system SYS may be implemented as a smartphone that is configured to function as the authentication apparatus 2 and the spoofing determination apparatus 3, as well as including the camera 1 and the stimulation apparatus 4. For example, the authentication system SYS may be implemented as a tablet personal computer that is configured to function as the authentication apparatus 2 and the spoofing determination apparatus 3, as well as including the camera 1 and the stimulation apparatus 4. In this case, it is possible to construct the authentication system SYS by using a relatively inexpensive and general-purpose apparatus, without requiring a special apparatus for performing the authentication operation and the spoofing determination operation.

### (2-2) Structure of Spoofing Determination Apparatus 3 in Second Example Embodiment

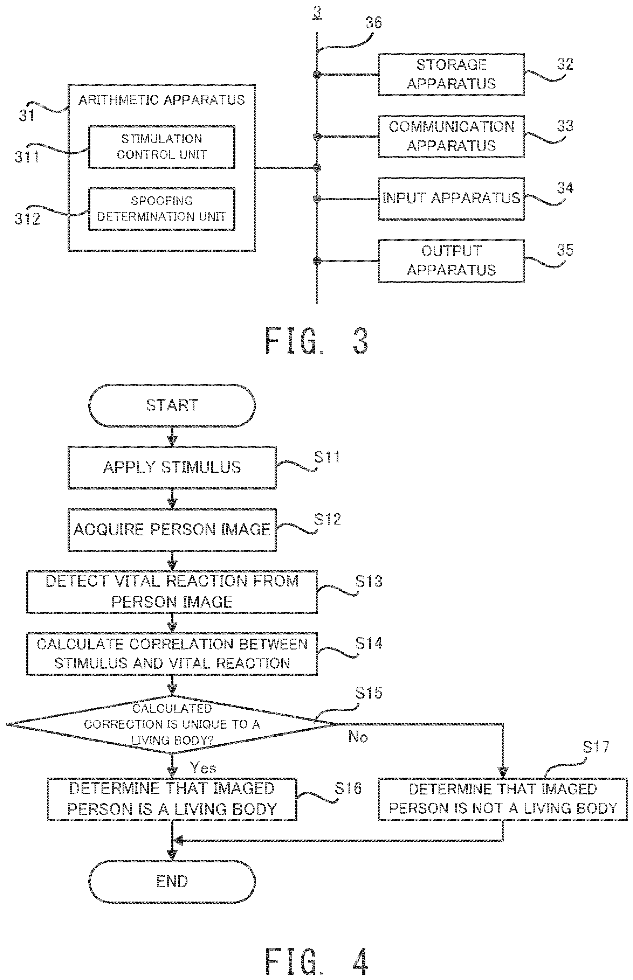

Next, a configuration of the spoofing determination apparatus 3 in the second example embodiment will be described with reference to FIG. 3. FIG. 3 is a block diagram illustrating the configuration of the spoofing determination apparatus 3 in the second example embodiment.

As illustrated in FIG. 3, the spoofing determination apparatus 3 includes an arithmetic apparatus 31, a storage apparatus 32, and a communication apparatus 33. Furthermore, the spoofing determination apparatus 3 may include an input apparatus 34 and an output apparatus 35. The spoofing determination apparatus 3, however, may not include at least one of the input apparatus 34 and the output apparatus 35. The arithmetic apparatus 31, the storage apparatus 32, the communication apparatus 33, the input apparatus 34, and the output apparatus 35 may be connected through a data bus 36.

The arithmetic apparatus 31 at least one of a CPU (Central Processing Unit), a GPU (Graphics Processing Unit), a FPGA (Field Programmable Gate Array), for example. The arithmetic apparatus 31 reads a computer program. For example, the arithmetic apparatus 31 may read a computer program stored in the storage apparatus 32. For example, the arithmetic apparatus 31 may read a computer program stored by a computer-readable and non-transitory recording medium, by using a not-illustrated recording medium reading apparatus provided in the spoofing determination apparatus 3. The arithmetic apparatus 31 may acquire (i.e., download or read) a computer program from a not-illustrated apparatus disposed outside the spoofing determination apparatus 3, through the communication apparatus 33 (or another communication apparatus). The arithmetic apparatus 31 executes the read computer program. Consequently, a logical functional block for performing an operation to be performed by the spoofing determination apparatus 3 (e.g., the spoofing determination operation) is realized or implemented in the arithmetic apparatus 31. That is, the arithmetic apparatus 31 is allowed to function as a controller for realizing or implementing the logical functional block for performing an operation (in other words, processing) to be performed by the spoofing determination apparatus 3.

FIG. 3 illustrates an example of the logical functional block realized or implemented in the arithmetic apparatus 31 to perform the authentication operation. As illustrated in FIG. 3, a stimulation control unit 311 that is a specific example of the "applying unit" and a spoofing determination unit 312 that is a specific example of the "determination unit" are realized or implemented in the arithmetic apparatus 31. The stimulation control unit 311 controls the stimulation apparatus 4 to apply to the imaged person the stimulus that induces the vital reaction of the imaged person captured in the person image IMG. The spoofing determination unit 312 determines whether or not the imaged person captured in the person image IMG is a living body on the basis of the correlation between the vital reaction of the imaged person captured in the person image IMG and the applied stimulus.

The storage apparatus 32 is configured to store desired data. For example, the storage apparatus 32 may temporarily store a computer program to be executed by the arithmetic apparatus 31. The storage apparatus 32 may temporarily store data that are temporarily used by the arithmetic apparatus 31 when the arithmetic apparatus 31 executes the computer program. The storage apparatus 32 may store data that are stored by the spoofing determination apparatus 3 for a long time. The storage apparatus 32 may include at least one of a RAM (Random Access Memory), a ROM (Read Only Memory), a hard disk apparatus, a magneto-optical disk apparatus, a SSD (Solid State Drive), and a disk array apparatus. That is, the storage apparatus 32 may include a non-transitory recording medium.

The communication apparatus 33 is configured to communicate with an apparatus external to the spoofing determination apparatus 3. For example, the communication apparatus 33 may be communicable with each of the camera 1 and the stimulation apparatus 4. In this instance, the stimulation control unit 311 may transmit a control signal for controlling the stimulation apparatus 4, to the stimulation apparatus 4 through the communication apparatus 33. The spoofing determination unit 312 may acquire the person image IMG from the camera 1 through the communication apparatus 32.

The input apparatus 34 is an apparatus that receives an input of information to the spoofing determination apparatus 3 from an outside of the spoofing determination apparatus 3. For example, the input apparatus 34 may include an operating apparatus (e.g., at least one of a keyboard, a mouse, and a touch panel) that is operable by an operator of the spoofing determination apparatus 3. For example, the input apparatus 34 may include a reading apparatus that is configured to read information recorded as data on a recording medium that can be externally attached to the spoofing determination apparatus 3.

The output apparatus 35 is an apparatus that outputs information to the outside of the spoofing determination apparatus 3. For example, the output apparatus 35 may output information as an image. That is, the output apparatus 35 may include a display apparatus (a so-called display) that is configured to display an image indicating the information that is desirably outputted. For example, the output apparatus 35 may output information as audio/sound. That is, the output apparatus 35 may include an audio apparatus (a so-called speaker) that is configured to output the audio/sound. For example, the output apparatus 35 may output information onto a paper surface. That is, the output apparatus 35 may include a print apparatus (a so-called printer) that is configured to desired information on the paper surface.

### (2-3) Spoofing Determination Operation in Second Example Embodiment

Next, with reference to FIG. 4, the spoofing determination operation performed by the spoofing determination apparatus 3 in the second example embodiment will be described. FIG. 4 is a flowchart illustrating a flow of the spoofing determination operation performed by the spoofing determination apparatus 3 in the second example embodiment.

As illustrated in FIG. 4, the stimulation control unit 311 controls the stimulation apparatus 4 to apply to the imaged person the stimulus that induces the vital reaction of the imaged person captured in the person image IMG (step S11). Consequently, the stimulation apparatus 4 applies to the imaged person the stimulus that induces the vital reaction of the imaged person captured in the person image IMG (step S11).

While the stimulus is being applied, the camera 1 images the imaging target range. Specifically, the camera 1 images the imaging target range at a predetermined imaging rate. For example, the camera 1 images the imaging target range at an imaging rate of imaging the imaging target range a desired number of times per second. Consequently, the camera 1 generates a plurality of person images IMG 1 corresponding to time series data. The camera 1 outputs the generated plurality of person images IMG to the spoofing determination apparatus 3.

The spoofing determination unit 312 acquires the plurality of person images IMG from the camera 1 (step S12). Thereafter, the spoofing determination unit 312 detects the vital reaction of the imaged person captured in each person image IMG on the basis of each person image IMG acquired in the step S12 (step S13).

The vital reaction may be any type of vital reaction as long as it is a reaction unique to a living body. The vital reaction may be any type of vital reaction as long as it is a reaction that rarely or does not occur in the imitation that is not a living body. For example, the vital reaction may include a reaction that occurs in the eye of a living body. An example of the reaction that occurs in the eye includes at least one of a dilated pupil and a contracted pupil. Another example of the reaction that occurs in the eye is a change in thickness of a crystalline lens (i.e., a change in focus of the crystalline lens). Another example of the reaction that occurs in the eye is a movement of a line of sight. Another example of the reaction that occurs in the eye is a movement of eyelids (typically, an eye blink). For example, the vital reaction may include a reaction that occurs in a body of a living body. An example of the reaction that occurs in the body is a movement of the face of a living body (e.g., a change in a face direction).

In the step S13, the spoofing determination unit 312 detects the vital reaction by calculating an index value of the vital reaction on the basis of the person image IMG. Typically, the index value of the vital reactions quantitatively indicates an extent of the vital reactions. For example, in order to detect the vital reaction including at least one of the dilated pupil and the contracted pupil, the spoofing determination unit 312 may calculate an index value indicating a diameter of the pupil. For example, in order to detect the vital reaction including the change in thickness of the crystalline lens, the spoofing determination unit 312 may calculate an index value indicating an emission direction of reflected light from the eye. For example, in order to detect the vital reaction including the movement of the line of sight, the spoofing determination unit 312 may calculate an index value indicating a direction of the line of sight. For example, in order to detect the vital reaction including the movement of the eyelids, the spoofing determination unit 312 may calculate an index value indicating a distance between an upper eyelid and a lower eyelid. For example, in order to detect the vital reaction including the movement of the face, the spoofing determination unit 312 may calculate an index value indicating a direction of the face.

In the step S13, the spoofing determination unit 312 may detect the vital reaction that occurs in a part of the imaged person to which the stimulus is applied in step S11. For example, when the stimulus is applied to the eye of the imaged person, the spoofing determination unit 312 may detect the vital reaction that occurs in the eye of the imaged person. Specifically, when the stimulus including at least one of light and a picture is applied to the eye of the imaged person, the spoofing determination unit 312 may detect the vital reaction that occurs in the eye of the imaged person. For example, when the stimulus is applied to an ear of the imaged person, the spoofing determination unit 312 may detect the vital reaction that occurs in the ear of the imaged person. Specifically, for example, when the stimulus including a sound is applied to the ear of the imaged person, the spoofing determination unit 312 may detect the vital reaction that occurs in the ear of the imaged person. In this instance, the spoofing determination unit 312 may be regarded as detecting a direct vital reaction in which the stimulus is propagated from a receptor to a central nerve and thus reflexively causes a reaction in the part that receives the stimulus.

In In the step S13, the spoofing determination unit 312 may detect the vital reaction that occurs in a different part from the part of the imaged person to which the stimulus is applied in the step S11. For example, when the stimulus is applied to the eye of the imaged person, the spoofing determination unit 312 may detect the vital reaction that occurs in a different part from the eye of the imaged person. Specifically, when the stimulus including at least one of light and a picture is applied to the eye of the imaged person, the spoofing determination unit 312 may detect the vital reaction that occurs in a different part from the eye of the imaged person (e.g., the movement of the face). For example, when the stimulus is applied to the ear of the imaged person, the spoofing determination unit 312 may detect the vital reaction that occurs in a different part from the ear of the imaged person. Specifically, for example, when the stimulus including a sound is applied to the ear of the imaged person, the spoofing determination unit 312 may detect the vital reaction (e.g., at least one of the movement of the eyelids, the movement of the line of sight, and the movement of the face) that occurs in a different part from the ear of the imaged person. In this case, the spoofing determination unit 312 may be regarded as detecting an indirect vital reaction in which the stimulus is propagated from a receptor to a central nerve, then passes through a complex process (e.g., a process that recalls some memory due to the stimulus), and causes a reaction in a different part from the part that receives the stimulus.

Thereafter, the spoofing determination unit 312 calculates the correlation between the vital reaction detected in the step S13 (especially, the index value of the vital reaction) and the stimulus applied in the step S11 (step S14). Then, it is determined whether or not the correlation between the vital reaction (especially, the index value of the vital reaction) and the stimulus, which is calculated in the step S14, is unique to a living body (step S15).

When the stimulus is applied and when the vital reaction of a stimulated living body is detected, the correlation between the stimulus applied to the living body and the vital reaction that occurs in the stimulated living body is often unique to the living body, and is determined depending on a type of the vital reaction and a type of the stimulus. For example, a time from when the stimulus is applied to a living body to when the vital reaction occurs in the living body due to the stimulus, is often unique to the living body, and is determined depending on the type of the vital reaction and the type of the stimulus. For example, a duration of the vital reaction in a living body due to the stimulus applied to the living body, is often unique to the living body, and is determined depending on the type of the vital reaction and the type of the stimulus. For example, a time from when the vital reaction occurs due to the stimulation in a living body to when the vital reaction starts to decline, is often unique to the living body, and is determined depending on the type of the vital reaction and the type of the stimulus. For example, an extent (in other words, a magnitude) of the vital reaction that occurs in a living body due to the stimulus applied to the living body, is often unique to the living body, and is determined depending on the type of the vital reaction and the type of the stimulus. For example, the vital reaction of a living body due to the stimulus applied to the living body, often changes (e.g., increases or decreases) in a change aspect that is unique to the living body and that is determined depending the type of the vital reaction and the type of the stimulus.

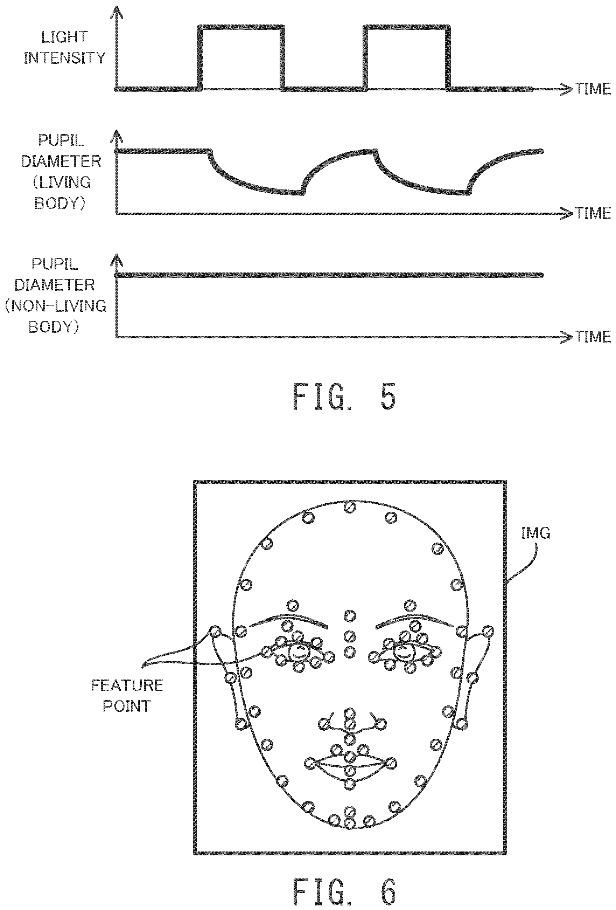

As an example, when the stimulus including light irradiated toward the eye of a living body is applied to the living body, the living body experiences the vital reaction of the contracted pupil such that an amount of light entering a retinal photoreceptor is less than or equal to an acceptable amount. Thereafter, when the application of the stimulus including the light irradiated toward the eye of the living body, is ended, the living body experiences the vital reaction of the dilated pupil. Therefore, as illustrated in FIG. 5, the diameter of the pupil calculated as the index value of the vital reaction from the person image IMG including the authentication subject, who is a living body, as the imaged person, starts to decrease within a certain time (e.g., a few milliseconds to a few seconds or a few minutes) after the stimulus is applied. The diameter of the pupil is reduced by a time constant corresponding to intensity of the light irradiated toward the eye, thereby converging toward a diameter corresponding to the intensity of the light irradiated toward the eye. Furthermore, the diameter of the pupil starts to increase within a certain time (e.g., a few milliseconds to several seconds or minutes) after the application of the stimulus is ended. The diameter of the pupil is increased by a time constant corresponding to the intensity of the light irradiated toward the eye, thereby converging toward a diameter corresponding to the intensity of environmental light. Furthermore, a size of the pupil diameter depends on the intensity of the light irradiated toward the eye. That is, there is a correlation unique to a living body illustrated in FIG. 5 between the vital reaction and the stimulus. On the other hand, the diameter of the pupil calculated as the index of the vital reaction on the basis of the person image IMG including the imitation, which is not a living body, as the imaged person, does not change even when the stimulus is applied, as illustrated in FIG. 5. That is, there is no correlation unique to the living body illustrated in FIG. 5 between the vital reaction and the stimulus.

Therefore, when the correlation between the vital reaction and the stimulus calculated in the step S 14 is unique to a living body, it is highly likely that the imaged person is a living body. That is, it is highly likely that the authentication subject who is a living body is captured as the imaged person in the person image IMG, and that the authentication subject does not impersonate another person. On the other hand, when the correlation between the vital reaction and the stimulus calculated in the step S14 is not unique to a living body, it is highly likely that the imaged person is not a living body. That is, it is highly likely that the imitation that is not a living body (i.e., the object that imitates the registered person who is different from the authentication subject) is captured as the imaged person in the person image IMG, and that the authentication subject impersonates another person. Therefore, when it is determined that the correlation between the vital reaction and the stimulus calculated in the step S14 is unique to a living body (the step S15: Yes), the spoofing determination unit 312 determines that the authentication subject is a living body (i.e., the authentication subject does not impersonate another person). On the other hand, when it is determined that the correlation between the vital reaction and the stimulus calculated in the step S14 is not unique to a living body (the step S15: No), the spoofing determination unit 312 determines that the authentication subject is not a living body (i.e., the authentication subject impersonates another person).

Information about the correlation that is unique to a living body and that is determined depending on the type of the vital reaction and the type of the stimulation (hereinafter referred to as "living body correlation information"), may be calculated in advance by using a result of an experiment or the like in which the stimulus is actually applied to each of many sample people and vital reactions of the stimulated sample people are actually detected. In this case, the spoofing determination apparatus 3 may previously store in the storage apparatus 32 the living body correlation information that is determined depending on the type of the vital reaction and the type of the stimulus. Specifically, the spoofing determination apparatus 3 may previously store in the storage apparatus 32 the living body correlation information that is determined depending on the type of the stimulus applied in the step S11 and the type of the vital reaction detected in the step S13. For example, when the stimulus including the light irradiated toward the eye of the imaged person is applied in the step S11 and when the vital reaction including the dilated pupil and the contracted pupil of the imaged person is detected in the step S13, the spoofing determination apparatus 3 may previously store in the storage apparatus 32 the living body correlation information that is calculated in advance by using the result of the experiment or the like in which the stimulus including light is actually applied to the eye of each of many sample people and the diameters of the pupils of the stimulated sample people are actually detected. As a result, the spoofing determination unit 312 is capable of properly determining whether or not the correlation between the vital reaction and the stimulus calculated in the step S14 is unique to a living body, on the basis of a detection result of the vital reaction in the step S13 and the living body correlation information previously stored in the storage apparatus 32.

The spoofing determination unit 312 may determine whether or not the correlation between the vital reaction and the stimulus calculated in the step S14 is unique to a living body on the basis of a degree of similarity between: the living body correlation information previously stored in the storage apparatus 32; and the correlation between the vital reaction and the stimulus calculated in the step S14. For example, when the living body correlation information previously stored in the storage apparatus 32 and the correlation calculated in the step S14 are so similar that the degree of similarity exceeds a predetermined determination threshold, the spoofing determination unit 312 may determine that the correlation calculated in the step S14 is unique to a living body. For example, when the correlation information stored in advance in the storage apparatus 32 and the correlation calculated in the step S14 are not so similar that the degree of similarity does not exceed the predetermined determination threshold, the spoofing determination unit 312 may determine that the correlation calculated in the step S14 is not unique to a living body.

The spoofing determination unit 312 may estimate the vital reaction that is estimated to be detected in the step S13 when the stimulus is applied in the step S11, on the basis of a stimulation aspect in the step S11 (e.g., at least one of a time at which the stimulus is applied and a degree of the applied stimulus) and the living body correlation information previously stored in the storage apparatus 32. That is, the spoofing determination unit 312 may estimate the correlation between the vital reaction and the stimulus that is estimated to be calculated in the step S14 when the stimulus is applied in the step S11. In this case, the spoofing determination unit 312 may determine whether or not the correlation between the vital reaction and the stimulation calculated in the step S14 is unique to a living body, on the basis of a degree of similarity between: the correlation between the vital reaction and the stimulation calculated in the step S14; and the estimated correlation between the vital reaction and the stimulus. For example, when the calculated correlation and the estimated correlation are so similar that the degree of similarity exceeds a predetermined determination threshold, the spoofing determination unit 312 may determine that the correlation calculated in the step S14 is unique to a living body. For example, when the calculated correlation and the estimated correlation are not so similar that the degree of similarity does not exceed the predetermined determination threshold, the spoofing determination unit 312 may determine that the correlation calculated in the step S14 is not unique to a living body.

### (2-4) Technical Effect of Authentication Systems SYS

As described above, the authentication system SYS in the second example embodiment (especially, the spoofing determination apparatus 3) actively applies to the imaged person (actually, the authentication subject) the stimulus that induces the vital reaction of the imaged person captured in the person image IMG, and determines whether or not the authentication subject impersonates another person on the basis of the correlations between the vital reaction of the imaged person captured in the person image IMG and the applied stimulus. Therefore, as described above, in a situation where the authentication subject impersonates another person by using the imitation (i.e., a duplicate of the biometric information about another person), the authentication system SYS is capable of properly determining that the authentication subject impersonates another person. In other words, the authentication system SYS is capable of properly preventing the authentication subject from impersonating another person by using the imitation (i.e., a duplicate of the biometric information about another person). It is because the imitation does not react to the actively applied stimulus. That is, the authentication system SYS is capable of solving a first technical problem of a possibility of erroneously authenticating the authentication subject who impersonates another person by using the imitation (i.e., a duplicate of the biometric information about another person).

In addition, there is a spoofing determination apparatus in a second comparative example that instructs the imaged person to move in a predetermined motion pattern and that determines the imaged person captured in the person image IMG is not a living body (i.e., the authentication subject impersonates another person) when the imaged person does not make the instructed particular motion. Since such a spoofing determination apparatus in the second comparative example generally instructs the imaged person to move in a motion pattern that is not related to the authentication operation, the authentication subject who intends to impersonate another person can easily identify the predetermined motion pattern used for the spoofing determination operation. Consequently, in a case where the authentication subject moves a robot that is an example of the imitation in the particular motion pattern, the spoofing determination apparatus in the second comparative example erroneously determines that the imaged person is a living body, which is a second technical problem. On the other hand, the authentication system SYS in the second example embodiment (especially, the spoofing determination apparatus 3) determines whether the imaged person is a living body on the basis of the vital reaction of the stimulated imaged person. That is, the authentication system SYS determines whether the imaged person is a living body on the basis of the vital reaction that unconsciously occurs in the imaged person, instead of a conscious (in other words, spontaneous) movement of the imaged person. Therefore, the authentication system SYS does not need to instruct the imaged person to move in the predetermined motion pattern in order to determine whether or not the authentication subject impersonates another person. That is, the authentication system SYS is capable of performing the spoofing determination operation, without making the authentication subject aware that the authentication system SYS is performing the spoofing determination operation. In this case, it is hard for the authentication subject to move the robot that is an example of the imitation in such a manner that the vital reaction changes in the aspect of correlating with the applied stimulus. Therefore, in a situation where the authentication subject impersonates another person by using the robot or the like that is an example of the imitation, the authentication system SYS is capable of properly determining that the authentication subject impersonates another person. In other words, the authentication system SYS is capable of properly preventing the authentication subject from impersonating another person by using the robot that is an example of the imitation. That is, the authentication system SYS is capable of solving the second technical problem of a possibility that the authentication subject impersonates another person by moving the robot in the particular motion pattern.

The authentication system SYS in the second example embodiment (especially, the spoofing determination apparatus 3) applies to the imaged person the stimulus including at least one of light, a picture, and a sound. In this case, the authentication system SYS may apply to the imaged person at least one of the light, the picture, and the sound, as an effect/performance of the authentication operation (or an effect/performance of some service). For example, the authentication systematic SYS may apply at least one of the picture and the sound for explaining the authentication operation, as the stimulus. As a result, the authentication system SYS is capable of applying the stimulus, without letting the authentication subject who intends to impersonate another person, know about the presence of the stimulus to be applied to the imaged person in order to perform the spoofing determination operation. That is, the authentication system SYS is capable of performing the spoofing determination operation, without making the authentication subject aware that the authentication system SYS is performing the spoofing determination operation. Consequently, it is even harder for the authentication subject to move the robot that is an example of the imitation in such a manner that the vital reaction changes in the aspect of correlating with the applied stimulus. Therefore, in a situation where the authentication subject impersonates another person by using the robot or the like that is an example of the imitation, the authentication system SYS is capable of more properly determining that the authentication subject impersonates another person. In other words, the authentication system SYS is capable of more properly preventing the authentication subject from impersonating another person by using the robot that is an example of the imitation.

In addition, the authentication system SYS is capable of performing the spoofing determination operations without using a special apparatus for performing the spoofing determination operation. For example, an example of a method for reliably preventing the authentication subject from impersonating another person by using the imitation (i.e., a duplicate of the biometric information about another person) as described above, is a method using a special apparatus for acquiring the biometric information used to authenticate the authentication subject. An example of the special apparatus is an apparatus using Magnetic Resonance Imaging (MRI), which is configured to generate a tomographic image of a living body that is an example of the biometric information. Such a special apparatus, however, leads to a significant cost increase in the authentication system SYS. The authentication system SYS, however, is capable of performing the spoofing determination operation by using the camera 1, which is a generally inexpensive, general-purpose apparatus. The authentication system SYS is capable of performing the spoofing determination operation by using at least one of a light irradiation apparatus, a display, and a speaker, which are generally inexpensive, general-purpose apparatuses, as the stimulation apparatus 4. Therefore, the authentication system SYS is capable of solving a third technical problem of a need of the special apparatus to prevent the authentication subject from impersonating another person.

In addition, the authentication system SYS in the second example embodiment (especially, the spoofing determination apparatus 3) determines whether or not the authentication subject impersonates another person, on the basis of not only the presence or absence of the vital reaction when the stimulus is applied, but also the correlation between the stimulus and the vital reaction. That is, the authentication system SYS determines whether the authentication subject impersonates another person, by determining whether or not the vital reaction changes in the aspect of correlating with the applied stimulus. For example, as described above, when the stimulus including the light irradiated toward the eye of the imaged person is applied in the step S11 and when the vital reaction including the dilated pupil and the contracted pupil of the imaged person is detected in the step S13, the authentication system SYS determines whether of not the authentication subject impersonates another person, by determining whether or not the pupil is dilated or contracted in the aspect of correlating with the applied stimulus, instead of simply determining whether or not the pupil is dilated or contracted. Therefore, as compared with a spoofing determination apparatus in a third comparative example that determines whether or not the authentication subject impersonates another person without using the correlation between the stimulus and the vital reaction, the authentication system SYS is capable of determining whether or not the authentication subject impersonates another person with high accuracy.

In addition, as described above, the authentication system SYS in the second example embodiment (especially, the spoofing determination apparatus 3) may determine whether or not the authentication subject impersonates another person on the basis of the vital reaction that occurs in a different part from the part of the imaged person to which the stimulus is applied in the step S11. In this case, the authentication system SYS determines whether or not the authentication subject impersonates another person, on the basis of an indirect vital reaction in which the stimulus is propagated from a receptor to a central nerve, then passes through a complex process (e.g., a process that recalls some memory due to the stimulus), and causes a reaction in a different part from the part that receives the stimulus. That is, the authentication system SYS is capable of determining whether or not the authentication subject impersonates another person, on the basis of the vital reaction that is hard to explain a direct causal relation with the applied stimulus. The vital reaction that is hard to explain a direct causal relation with the applied stimulus, is hard to be disguised for the authentication subject who intends to impersonate another person. Therefore, the authentication system SYS is capable of properly determining whether or not the authentication subject impersonates another person. In other words, the authentication system SYS is capable of properly preventing the authentication subject from impersonating another person.

### (2-5) Examples of Spoofing Determination Operation

Next, specific examples of the spoofing determination operation will be described.

### (2-5-1) First Specific Example of Spoofing Determination Operation

First, the spoofing determination operation in a first specific example of will be described. The spoofing determination operation in the first specific example embodiment is a spoofing determination operation in which the stimulus that induces the movement of the eyelids of the imaged person is applied to the imaged person in the step S11 in FIG. 4, and in which the vital reaction including the movement of the eyelids (i.e., the eye blink) of the imaged person is detected in step S13 in FIG. 4.

The stimulus that induces the movement of the eyelids may include a stimulus applied directly to an eyeball. The stimulus applied directly to the eyeball may include at least one of a stimulus of contacting the eyeball and a stimulus of wind blowing to the eyeball. When such a stimulus applied directly to the eyeball is applied to a living body, a human (i.e., a living body, and the same shall apply hereinafter) is likely to blink to protect the eyeball. Therefore, the stimulus applied directly to the eyeball is usable as the stimulus that induces the movement of the eyelids.

The stimulus that induces the movement of the eyelids may include light irradiated to the eye. When the light is irradiated to the eye, a human is likely to feel glare and blink. Therefore, the light irradiated to the eye is usable as the stimulus that induces the movement of the eyelids. For a similar reason, the stimulus that induces the movement of the eyelids may include a picture that causes a human to feel glare.

When a human recognizes an action performed by another living body (e.g., a human or an animal), the human tends to sympathize the action and make the same action in synchronization. Therefore, the stimulus that induces the movement of the eyelids may include a picture that causes another living body to blink. Consequently, a human who recognizes the picture that that causes another living body to blink, is likely to blink in sympathy with the eye blink of another living body. Therefore, the picture that causes another living body to blink is usable as the stimulus that induces the movement of the eyelids.

A human in tension is likely to blink as an indirect reaction to the tension. A human in tension is likely to blink more frequently than a human not in tension. The number of continuous blinks of a human in tension, may be more than that of a human not in tension. A speed of the movement of the eyelids when a human in tension blinks, may be higher than that of the movement of the eyelids when a human not in tension blinks. Therefore, the stimulus that induces the movement of the eyelids may include a stimulus that increases human tension, as compared with that before the stimulus is applied.

The stimulus that increases human tension may include a picture that increases human tension. An example of the picture that increases human tension includes at least one of a picture in which a rapidly approaching object is captured, a picture in which scenery seen by a human looking down from a high altitude is captured, a picture in which a sharp knife is captured, a picture in which cars (or motorcycles, etc.) participating in racing are captured, and a picture in which a living body in danger is captured.

The stimulus that increases human tension may include a sound that increases human tension. An example of the sound that increases human tension includes at least one of a wind noise (i.e., a noise caused by airflow), a sound that decreases in volume while changing from a high pitch to a low pitch, an impulsive sound (or a sound with high sharpness), and a scream of a living body.

The stimulus that increases human tension may include a task that increases human tension. An example of the task that increases human tension includes at least one of a task requiring memory recall (e.g., a task requiring an answer within a time limit after presentation of a question requiring memory recall), a task requiring calculation (e.g., a task requiring calculation to be completed within a time limit), a game (e.g., a game competing for scores with others), and a quiz (e.g., a quiz competing for the number of correct answers with others). Such a task may require a human assigned a task to perform active work, and it is thus possible to increase human tension more efficiently. In addition, the game or quiz may solve the above-described second technical problem in that it can be applied as an effect/performance of the authentication operation (or an effect/performance of some service) as described above. Such a task may be assigned/applied as a picture or a sound that explains the contents of the task and that requests the execution of the task.

The task requiring memory recall may include a task requiring memory recall of confidential information that is only known to a human performing the task. In this case, the confidential information may be registered in advance in the spoofing determination apparatus 3, and the spoofing determination apparatus 3 may apply the task requiring memory recall of the confidential information that is only known to the human performing the task, as the stimulus that increases human tension, on the basis of the confidential information registered in advance. Similarly, the task requiring calculation may include a task requiring calculation using the confidential information that is only known to the human performing the task. In this case, the confidential information may be registered in advance in the spoofing determination apparatus 3, and the spoofing determination apparatus 3 may assign/apply the task requiring calculation using the confidential information that is only known to the human performing the task (e.g., addition using a numerical value indicated by the confidential information, etc.), as the stimulus that increases human tension, on the basis of the numerical value of the confidential information registered in advance.

When such confidential information is used, the authentication apparatus 1 may authenticate the authentication subject on the basis of the biometric information, as well as allowing the authentication subject to input the confidential information and authenticating the authentication subject on the basis of the inputted confidential information. That is, the authentication apparatus 1 may perform multi-factor authentication.

A human in tension is likely to blink from a sense of security when the tension is relieved. Therefore, the stimulus that induces the movement of the eyelids may include a stimulus that relieves human tension more than before the stimulus is applied. In particular, the stimulus that induces the movement of the eyelids may include a first stimulus that increases human tension and a second stimulus that is added after the first stimulus is applied and that relieves human tension. In this case, the spoofing determination apparatus 3 may apply the first stimulus (e.g., a picture, a sound or a task) that increases human tension, and then may apply the second stimulus that relieves human tension.

The stimulus that relieves human tension may include a picture that relieves human tension. An example of the picture that relieves human tension includes at least one of a picture encouraging a viewer to take a rest, a picture in which a luxury item (e.g., at least one of tea, coffee, and cigarette) is captured, a picture in which an object used for a rest (e.g., at least one of a bed and sofa) is captured, and a picture in which natural scenery (e.g., at least one of a sea, a mountain, and a grassland) is captured.

The stimulus that relieves human tension may include a sound that relieves human tension. An example of the sound that relieves human tension includes at least one of a natural sound (e.g., at least one of a wave sound and a forest sound), classic music, and a breathing sound of a baby.

When the stimulation control unit 311 applies the stimulus that induces the movement of the eyelids, the spoofing determination unit 312 detects the vital reaction including the eye blink of the imaged person captured in the person image IMG (step S13 in FIG. 4). For example, when the imaged person blinks, a distance between the upper eyelid and the lower eyelid of the imaged person changes. Therefore, the spoofing determination unit 312 may calculate the distance between the upper eyelid and the lower eyelid of the imaged person (hereinafter referred to as an "eye size"), as the index value of the vital reaction including the eye blink. Specifically, the spoofing determination unit 312 may extract feature points (in other words, landmarks) of each of the upper and lower eyelids of the imaged person on the basis of the person image IMG. An example of the respective feature points of the upper and lower eyelids of the imaged person is conceptually illustrated in FIG. 6. FIG. 6 illustrates the feature points of the face of the imaged person including the respective feature points of the upper and lower eyelids of the imaged person. Thereafter, the spoofing determination unit 312 may calculate the eye size, on the basis of positions of the respective feature points of the upper and lower eyelids of the imaged person. As described above, the spoofing determination unit 312 is capable of relatively easily detecting the vital reaction including the eye blink, without using a special apparatus.

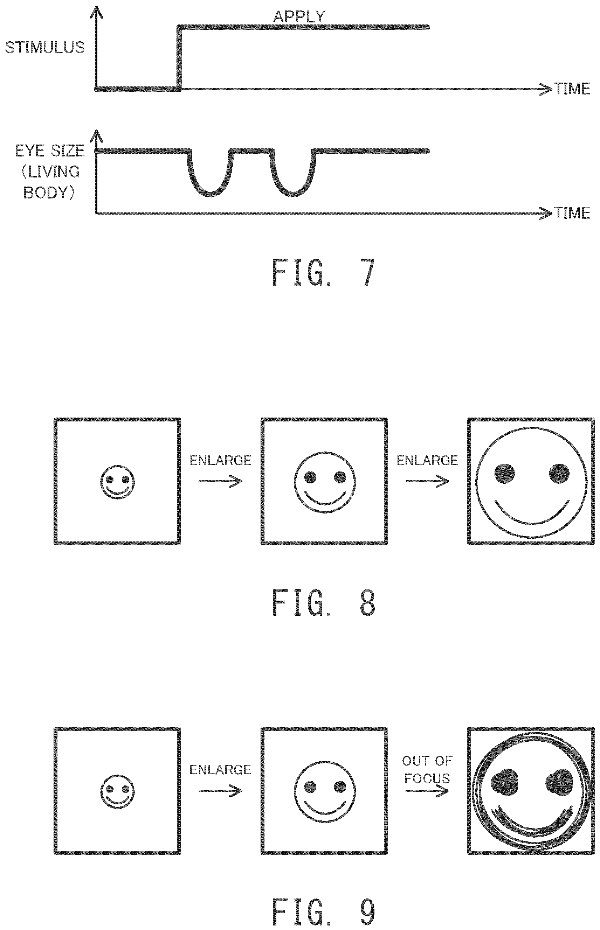

Thereafter, the spoofing determination unit 312 calculates the correlation between the vital reaction including the eye blink and the stimulus (step S14 in FIG. 4). For example, the spoofing determination unit 312 calculates a correlation between the eye size and the stimulus. FIG. 7 illustrates an example of the correlation between the eye size and the stimulus, calculated by the determination unit 312, when the stimulus that induces the eye blink is actually added to the authentication subject who is a living body. As illustrated in FIG. 7, when the imaged person (i.e., a human) blinks, the eye size of the authentication subject decreases and then increases. Therefore, the spoofing determination unit 312 is capable of detecting the vital reaction including the eye blink by calculating the eye size. FIG. 7 illustrates a case where the authentication subject blinks twice within a certain period. The spoofing determination unit 312 determines whether or not the authentication subject impersonates another person, by determining whether or not the correlation between the blink and the stimulus illustrated in FIG. 7 is unique to a living body. For example, the authentication subject is likely to blink at a time unique to a living body, which is determined in accordance with a time when the stimulus is applied to the authentication subject. For example, the authentication subject is likely to blink at a speed unique to a living body, which is determined in accordance with the stimulus applied to the authentication subject. As an example, a human is likely to blink due to properties unique to a living body such that a speed of closing the eyelids is higher than a speed of opening the eyelids. For example, the authentication subject is likely to blink at a frequency unique to a living body, which is determined in accordance with the stimulus applied to the authentication subject. Therefore, the spoofing determination unit 312 may use at least one of information about a time of the eye blink, information about a speed of moving the eyelids due to the eye blink, and information about a frequency of the eye blink, as information indicating the correlation between the eye blink and the stimulus, thereby determining whether or not the calculated correlation between the blink and the stimulus is unique to a living body. Consequently, the spoofing determination unit 312 is capable of properly determining whether or not the authentication subject impersonates another person.

### (2-5-2) Second Specific Example of Spoofing Determination Operation

First, the spoofing determination operation in a second specific example will be described. The spoofing determination operation in the second example embodiment is a spoofing determination operation in which the stimulus that induces adjustment of focus of the crystalline lens of the eye of the imaged person is applied to the imaged person in the step S11 in FIG. 4, and in which the vital reaction including a reaction of adjusting the focus of the crystalline lens of the eye of the imaged person is detected in the step S13 in FIG. 4.

The crystalline lens adjusts the focus by changing the thickness of the crystalline lens. Therefore, the stimulus that induces the adjustment of the focus of the crystalline lens may be considered to be substantially equivalent to a stimulus that changes the thickness of the crystalline lens. Similarly, the vital reaction including the reaction of adjusting the focus of the crystalline lens may be considered to be substantially equivalent to a vital reaction including a reaction of changing the thickness of the crystalline lens.

The focus of the crystalline lens of a human is adjusted in accordance with a distance from the human (especially, the human eyes) to an object viewed by the human. Therefore, the stimulus that induces the adjustment of the focus of the human crystalline lens may include a stimulus that causes a human to visually recognize an object moving back and forth with respect to the human.

In order to apply the stimulus that causes a human to visually recognize the object moving back and forth with respect to the human, however, the authentication system SYS needs a special apparatus for moving the object. Furthermore, when an operation of moving the object is not related to the authentication movement, the authentication subject who intends to impersonate another person can easily identify that the object is moving for the spoofing determination operation. On the other hand, a human grasps the position of the object in a three-dimensional space, by using information about enlargement or reduction of the size of the object caused by the movement of the object in a longitudinal direction with respect to the human. In addition, a human grasps the position of the object in the three-dimensional space, by using information about a sound emitted by the object. Therefore, the stimulus that induces the adjustment of the focus of the human crystalline lens may include at least one of a picture and a sound that indirectly induces the adjustment of the focus of the human crystalline lens.

The picture that indirectly induces the adjustment of the focus of the human crystalline lens may include a picture that induces an illusion in stereoscopic vision of a human, thereby indirectly inducing the adjustment of the focus of the crystalline lens. An example of the picture that induces an illusion in the stereoscopic vision of a human, thereby indirectly inducing the adjustment of the focus of the crystalline lens, is a picture illustrating an object that is gradually enlarged (or gradually reduced) such that the human can grasp it as the object moving back and forth, as illustrated in FIG. 8. In this case, since the human has an illusion that the object is gradually approaching the human or the object is gradually moving away from the human, the focus of the human crystalline lens is adjusted. Another example of the picture that induces an illusion in the stereoscopic vision of a human, thereby indirectly inducing the adjustment of the focus of the crystalline lens, may include a picture illustrating an object that is enlarged (or reduced) and then becomes out of focus, as illustrated in FIG. 9. That is, another example of the picture that induces an illusion in the stereoscopic vision of a human, thereby indirectly inducing the adjustment of the focus of the crystalline lens, may include a picture illustrating an object that is out of focus during enlargement (or reduction). The object out of focus may be a blurred object. In this situation, since the human has an illusion that the eyes are not properly focused, the focus of the human crystalline lens is adjusted.

The sound that indirectly induces the adjustment of the focus of the human crystalline lens may include a sound that induces an illusion in the stereoscopic vision of a human, thereby indirectly inducing the adjustment of the focus of the crystalline lens. An example of the sound that induces an illusion in the stereoscopic vision of a human, thereby indirectly inducing the adjustment of the focus of the crystalline lens, is a sound of the object that is gradually approaching the human or that is gradually away from the human. In this case, since the human has an illusion that the object is gradually approaching the human or the object is gradually moving away from the human, the focus of the human crystalline lens is adjusted.

The sound that indirectly induces the adjustment of the focus of the human crystalline lens may be applied with the picture that indirectly induces the adjustment of the focus of the human crystalline lens. In this case, since the human is more likely to have an illusion that the object is gradually approaching the human or the object is gradually moving away from the human, the focus of the human crystalline lens is more easily adjusted. In this case, at least one of a sound with increased volume, a sound with raised pitch, a sound with increased sharpness, and a sound with increased roughness may be used as the sound that indirectly induces the adjustment of the focus of the human crystalline lens.

When the stimulation control unit 311 applies the stimulus that induces the adjustment of the focus of the crystalline lens, the spoofing determination unit 312 detects the vital reaction including the reaction of adjusting the focus of the crystalline lens of the imaged person captured in the person image IMG (step S13 in FIG. 4). It is, however, not always easy to directly detect the reaction of adjusting the focus of the crystalline lens on the basis of the person image IMG. Specifically, since the reaction of adjusting the focus of the crystalline lens is equivalent to the reaction of changing the thickness of the crystalline lens as described above, it is not always easy to directly detect the thickness of the crystalline lens itself on the basis of the person image IMG. Therefore, the spoofing determination unit 312 may calculate an index value indirectly indicating the reaction of adjusting the focus of the crystalline lens, on the basis of the person image IMG.

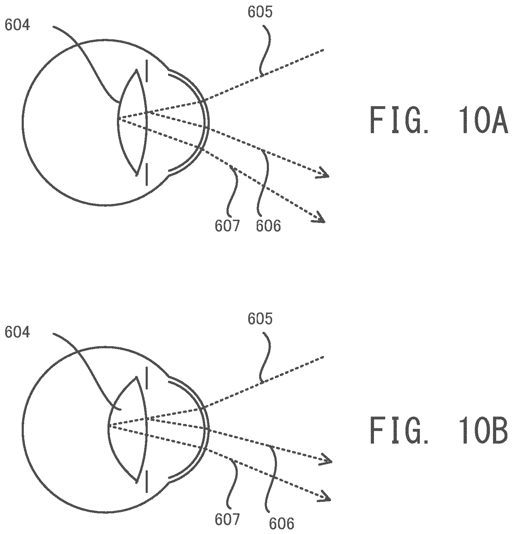

As an example, when the focus of the crystalline lens is adjusted (i.e., the thickness of the crystalline lens changes), an emission direction of reflected light emitted from the crystalline lens on which incident light is incident (i.e., reflected light of the incident light) changes. For example, FIG. 10A is a cross-sectional view illustrating reflected light 606 and 607 emitted from a relatively thin crystalline lens 604, and FIG. 10B is a cross-sectional view illustrating reflected light 606 and reflected light 607 emitted from a relatively thick crystalline lens 604. As illustrated in FIG. 10A and FIG. 10B, incident light 605 entering the crystalline lens 604 is reflected by each of a front surface and a back surface of the crystalline lens 604. As a result, the reflected light 606 reflected by the front surface of the crystalline lens 604 and the reflected light 607 reflected by the back surface of the crystalline lens 604 are emitted from the crystalline lens 604. When a human sees a nearby object, the crystalline lens 604 is thicker than that when the human sees an object in the distance. Thus, FIG. 10A including the relatively thin crystalline lens 604, illustrates the crystalline lens 604 of the human seeing the object in the distance, and FIG. 10B including the relatively thick crystalline lens 604, illustrates the crystalline lens 604 of the human seeing the nearby object. As illustrated in FIG. 10A and FIG. 10B, the change in the thickness of the crystalline lens 604 changes the emission directions of the reflected light 606 and the reflected light 607. It is because the change in the thickness of crystalline lens 604 results in a change in radius of curvature of the front surface and the back surface of the crystalline lens 604. Therefore, the emission directions of the reflected light 606 and the reflected light 607 from the crystalline lens 604 are usable as the index value indirectly indicating the reaction of adjusting the focus of the crystalline lens. Therefore, the spoofing determination unit 312 may calculate the emission direction of at least one of the reflected light 606 and the reflected light 607 from the crystalline lens 604, on the basis of the person image IMG.

In order to calculate the emission direction of the reflected light from the crystalline lens, the authentication system SYS may enter the incident light into the crystalline lens from a point light source (or a picture that imitates the point light source). In this case, an image formed by the reflected from the crystalline lens is referred to as a Purkinje-Sanson mirror image. In particular, the image formed by the reflected light from the front surface of the crystalline lens is referred to as a Purkinje-Sanson mirror image P3, and the image formed by the reflected light from the back surface of the crystalline lens is referred to as a Purkinje-Sanson mirror image P4. Therefore, the spoofing determination unit 312 may detect the Purkinje-Sanson mirror images P3 and P4 on the basis of the person image IMG, and may calculate the emission direction of the reflected light from the crystalline lens on the basis of positions of the detected Purkinje-Sanson mirror images P3 and P4.

The spoofing determination unit 312 may calculate an angle formed by an axis extending along a propagation direction of the reflected light from the crystalline lens and a predetermined reference axis, as the emission direction of the reflected light from the crystalline lens. That is, the spoofing determination unit 312 may calculate a rotation angle of the axis extending along the propagation direction of the reflected light from the crystalline lens based on the predetermined reference axis, as the emission direction of the reflected light from the crystalline lens. This angle may be referred to as an emission angle.

The reflected light from the crystalline lens is light with extremely low intensity. Therefore, in order to facilitate detection of reflected light from the crystalline lens (e.g., detection of the Purkinje-Sanson mirror images P3 and P4), the incident light that flashes or changes in brightness (i.e., modulated incident light) may enter the crystalline lens. In this case, as in a lockin amplifier or heterodyne detection, the spoofing determination unit 312 may detect the reflected light from the crystalline lens by extracting a picture component synchronized with modulation.

The incident light entering the crystalline lens is also reflected by each of the front and back surfaces of a cornea. In this instance, an image formed by the reflected light from the front surface of the cornea is referred to as a Purkinje-Sanson mirror image P1, and an image formed by the reflected light from the back surface of the cornea is referred to as a Purkinje-Sanson mirror image P2. In this case, the spoofing determination unit 312 may calculate the Purkinje-Sanson mirror images P1 and P2 on the basis of the person image IMG, and may calculate the emission direction of the reflected light from the crystalline lens on the basis of positions of the calculated Purkinje-Sanson mirror images P1 and P2. The cornea, however, hardly reacts (e.g., deforms) due to the stimulus, unlike the crystalline lens. Furthermore, since the cornea can be easily duplicated by an artificial eye or the like, a malicious authentication subject can easily impersonate another person by using the imitation that imitates the cornea of another person (e.g., an artificial eye). Therefore, in order to determine whether or not the authentication subject impersonates another person, the spoofing determination unit 312 may not use the emission direction of the reflected light from the cornea. That is, the spoofing determination unit 312 may not calculate the emission direction of the reflected light from the cornea, in order to determine whether or not the authentication subject impersonates another person.

Then, the spoofing determination unit 312 calculates a correlation between the emission direction of the reflected light from the crystalline lens (i.e., the index value indicating the reaction of adjusting the focus of the crystalline lens) and the stimulus (step S14 in FIG. 4). The spoofing determination unit 312 determines whether or not the authentication subject impersonates another person, by determining whether or not the calculated correlation between the emission direction of the reflected light and the stimulus is unique to a living body. Consequently, the spoofing determination unit 312 is capable of properly determining whether or not authentication subject impersonates another person.

### (2-5-3) Third Specific Example of Spoofing Determination Operation

Next, the spoofing determination operation in a third specific example will be described. The spoofing determination operation in the third example embodiment is a spoofing determination operation in which the stimulus that induces the respective movements of the face and the line of sight of the imaged person is applied to the imaged person in the step S11 in FIG. 4, and in which the vital reaction including the respective movements of the face and the line of sight of the imaged person is detected in the step S13 in FIG. 4.

A human usually moves the face and the line of sight with the awareness of moving the face and the line of sight. The human face and the line of sight, however, may be unconsciously moved by the stimulus externally applied to the human. For example, the human face and the line of sight may be moved unconsciously in reaction to light. For example, the human face and the line of sight may be unconsciously moved in reaction to an explosion or screaming. Therefore, the stimulus that induces the respective movements of the face and the line of sight may include a stimulus that induces the respective movements of the face and the line of sight without making a human aware. The stimulus that induces the respective movements of the face and the line of sight, however, may include a stimulus that consciously induces a human to move the face and the line of sight.

The stimulus that induces the respective movements of the face and the line of sight may include light irradiated to a human from a predetermined direction. In this case, the human is likely to move the face and the line of sight to face in the predetermined direction due to the light irradiated to the human from the predetermined direction. That is, the human is likely to estimate a direction in which the light is irradiated by the stereoscopic vision, and is likely to move the face and the line of sight to face in the estimated direction. Therefore, the light irradiated to the human from the predetermined direction is usable as the stimulus that induces the respective movements of the face and the line of sight.

The stimulus that induces the respective movements of the face and the line of sight may include a picture displayed to a human from a predetermined direction. In this case, the human is likely to move the face and the line of sight to face in the predetermined direction due to the picture displayed to the human from the predetermined direction. That is, the human is likely to estimate a direction in which the picture is displayed, by the stereoscopic vision, and is likely to move the face and the line of sight to face in the estimated direction. Therefore, the picture displayed to the human from the predetermined direction is usable as the stimulus that induces the respective movements of the face and the line of sight.

The picture displayed to the human from the predetermined direction may include a picture for directing a human who notices the picture in a particular direction, without clearly indicating a direction in which the human who notices the picture should face. For example, the picture displayed to the person from the predetermined direction may include a picture including a message of "Please look over here." In this case, it is even harder to move the face and the line of sight in an aspect of correlating with the displayed picture, by using the robot that is an example of the imitation. Therefore, the spoofing determination apparatus 3 is capable of more properly preventing the authentication subject from impersonating another person by using the robot that is an example of the imitation.

The stimulus that induces the respective movements of the face and the line of sight may include a sound outputted toward a human from a predetermined direction. In this case, the human is likely to move the face and the line of sight to face in the predetermined direction due to the sound outputted toward the human from the predetermined direction. That is, the human is likely to estimate a direction in which the sound is outputted, by stereophonic sound, and is likely to move the face and the line of sight to face in the estimated direction. Therefore, the sound outputted toward the human from the predetermined direction is usable as the stimulus that induces the respective movements of the face and the line of sight.

The sound outputted toward the human from the predetermined direction may include a sound for directing a human who hears the sound in a particular direction, without clearly indicating a direction in which the human who hears the sound should face. For example, the sound outputted toward the human from the predetermined direction may include a sound corresponding to an instruction of "Please look over here." In this case, it is even harder to move the face and the line of sight in an aspect correlating with the outputted sound, by using the robot that is an example of the imitation. Therefore, the spoofing determination apparatus 3 is capable of more properly preventing the authentication subject from impersonating another person by using the robot that is an example of the imitation.

The predetermined direction in which the stimulus that induces the respective movements of the face and the line of sight is irradiated, may be a direction in which the camera 1 exists when viewed from the authentication subject who is an imaging target of the camera 1. In this case, light may be emitted from the direction in which the camera 1 exists, as the stimulus that induces the respective movements of the face and the line of sight. Similarly, the predetermined direction in which the picture that is the stimulus that induces the respective movements of the face and the line of sight is displayed, may be the direction in which the camera 1 exists when viewed from the authentication subject who is the imaging target of the camera 1. In this case, the picture may be displayed from the direction in which the camera 1 exists, as the stimulus that induces the respective movements of the face and the line of sight. Similarly, the predetermined direction in which the sound that is the stimulus that induces the respective movements of the face and the line of sight is outputted, may be the direction in which the camera 1 exists when viewed from the authentication subject who is the imaging target of the camera 1. In this case, the sound may be outputted from the direction in which the camera 1 exists, as the stimulus that induces the respective movements of the face and the line of sight. In this situation, the authentication subject is typically likely to move the face and the line of sight to face in the direction in which the camera 1 exists. Here, since the camera 1 images the authentication subject to perform the authentication operation, an action of moving the face and the line of sight to face in the direction in which that the camera 1 exists, is a natural action performed by the authentication subject during the authentication operation. Therefore, the spoofing determination apparatus 3 is capable of performing the spoofing determination operation without making the authentication subject recognize that the spoofing determination apparatus 3 is performing the spoofing determination operation. Furthermore, since the spoofing determination apparatus 3 is capable of moving the face and the line of sight of the authentication subject to face in the direction in which the camera 1 exists, the camera 1 is capable of properly imaging the authentication subject in the authentication operation.

When the stimulation control unit 311 applies the stimulus that induces movements of the face and the line of sight, the spoofing determination unit 312 detects the vital reaction including the movements of the face and the line of sight of the imaged person captured in the person image IMG (step S13 in FIG. 4). For example, the movement of the face of the imaged person changes a direction in which the face of the imaged person is directed. Similarly, for example, the movement of the line of sight of the imaged person changes a direction in which the line of sight of the imaged person is directed. Therefore, the spoofing determination unit 312 may calculate (i.e., detect) the direction in which the face of the imaged person is directed, and the direction in which the line of sight of the imaged person is directed. In this case, for example, the spoofing determination unit 312 may calculate the direction in which the face of the imaged person is directed, by using an existing method for calculating the direction in which the face of a human is directed. As an example, the spoofing determination unit 312 may extract feature points (in other words, landmarks) of the face of the imaged person on the basis of the person image IMG, and may calculate the direction of the face on the basis of the extracted feature points. Similarly, for example, the spoofing determination unit 312 may calculate the direction in which the line of sight of the imaged person is directed, by using an existing method for calculating the direction in which the line of sight of the person is directed. As an example, the spoofing determination unit 312 may extract feature points (in other words, landmarks) of the eye(s) of the imaged person on the basis of the person image IMG, and may calculate the direction of the line of sight on the basis of the extracted feature points. As described above, the spoofing determination unit 312 is capable of relatively easily detecting the vital reaction including the movements of the face and the line of sight, without using a special apparatus.

The spoofing determination unit 312 may calculate an angle (a line-of-sight angle) formed by a line-of-sight axis extending along the direction in which the line of sight of a human is directed and a predetermined reference axis, as the direction in which the line of sight of the human is directed. That is, the spoofing determination unit 312 may calculate a rotation angle (a line-of-sight angle) of the line-of-sight axis based on the predetermined reference axis, as the direction in which the line of sight of the human is directed. For example, the spoofing determination unit 312 may calculate a rotation angle of the line-of-sight axis in a pan direction that is a rotation direction around a vertical axis. For example, the spoofing determination unit 312 may calculate a rotation angle of the line-of-sight axis in a roll direction that is a rotation direction around an axis extending along a longitudinal direction of the human. For example, the spoofing determination unit 312 may calculate a rotation angle of the line-of-sight axis in a tilt direction that is a rotation direction around an axis extending along a lateral direction of the human.

The spoofing determination unit 312 may calculate an angle (a face angle) formed by a face axis extending along the direction in which the face of a human is directed and a predetermined reference axis, as the direction in which the face of the human is directed. That is, the spoofing determination unit 312 may calculate a rotation angle of the face axis based on the predetermined reference axis, as the direction in which the face of the human is directed. For example, the spoofing determination unit 312 may calculate a rotation angle of the face axis in the pan direction that is a rotation direction around the vertical axis. For example, the spoofing determination unit 312 may calculate a rotation angle of the face axis in the roll direction is a rotation direction around the axis extending along the longitudinal direction of the human. For example, the spoofing determination unit 312 may calculate a rotation angle of the face axis in the tilt direction that is a rotation direction around the axis extending along the lateral direction of the human.

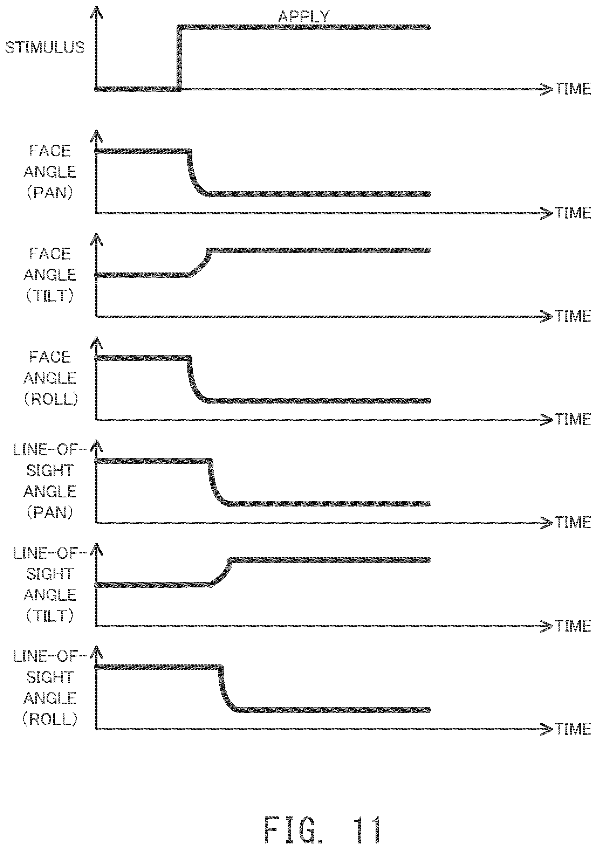

Thereafter, the spoofing determination unit 312 calculates the correlation between the vital reaction and the stimulus including the movements of the face and the line of sight (step S14 in FIG. 4). For example, the spoofing determination unit 312 calculates a correlation between each of the direction in which the face is directed and the direction in which the line of sight is directed, and the stimulus. FIG. 11 illustrates an example of the correlation between each of the direction in which the face is faced (the face angle) and the direction in which the line of sight is directed (the line-of-sight angle), which are calculated by the determination unit 312, and the stimulus, when the stimulus that induces the movements of the face and the line of sight is actually added to the authentication subject who is a living body. As illustrated in FIG. 11, when the stimulated imaged person moves the face, the face angle of the authentication subject changes. Similarly, when the stimulated imaged person moves the line of sight, the line of sight of the authentication subject changes. The spoofing determination unit 312 determines whether or not the authentication subject impersonates another person, by determining whether or not the correlation between each of the direction in which the face is directed and the direction in which the line of sight is directed, and the stimulus, illustrated in FIG. 11, is unique to a living body. Consequently, the spoofing determination unit 312 is capable of properly determining whether or not the authentication subject impersonates another person.

Here, as can be seen from FIG. 11, when the authentication subject intends to impersonate another person by using the robot that is an example of the imitation, the authentication subject needs to change the face angle of the robot in each of the pan direction, the tilt direction, and the roll direction, and the line-of-sight angle of the robot in each of the pan direction, the tilt direction, and the roll direction, in synchronization with each other in accordance with the applied stimulus. It is, however, extremely hard for the authentication subject to control the robot to imitate all the three types of line-of-sight angles and the three types of face angles. Therefore, the spoofing determination apparatus 3 is capable of more properly preventing the authentication subject from impersonating another person by using the robot that is an example of the imitation.

### (2-6) Modified Example

In the above description, the authentication system SYS includes the authentication apparatus 1 and the spoofing determination apparatus 3 as separate apparatuses. The authentication system SYS, however, may include a single apparatus that functions as the authentication apparatus 1 and that also functions as the spoofing determination apparatus 3. That is, the authentication apparatus 1 may be integrated with the spoofing determination apparatus 3.

### (3) Supplementary Notes

With respect to the example embodiment described above, the following Supplementary Notes are further disclosed.

### [Supplementary Note 1]

An information processing apparatus including: an applying unit that applies a stimulus including at least one of light, a picture, and a sound that induces a change in a vital reaction of a target person; and
a determination unit that determines whether or not the target person is a living body, on the basis of a correlation between the stimulus and the vital reaction that occurs in the target person to which the stimulus is applied.

### [Supplementary Note 2]

The information processing apparatus according to Supplementary Note 1, wherein
the applying unit applies the stimulus to a first part of the target person, and
the correlation includes a correlation between the stimulus and the vital reaction in a second part of the target person that is different from the first part to which the stimulus is applied.

### [Supplementary Note 3]

The information processing apparatus according to Supplementary Note 1 or 2, wherein the vital reaction includes an eye blink.

### [Supplementary Note 4]

The information processing apparatus according to Supplementary Note 3, wherein
the applying unit applies the stimulus by outputting a picture of a living body blinking, toward the target person, and
the correlation includes a correlation between the stimulus and at least one of a time of the eye blink and a frequency of the eye blink.

### [Supplementary Note 5]

The information processing apparatus according to Supplementary Note 3 or 4, wherein
the applying unit applies the stimulus by outputting toward the target person at least one of a picture and a sound that increases tension of the target person, and
the correlation includes a correlation between the stimulus and at least one of a time of the eye blink, a speed of moving eyelids by the eye blink, and a frequency of the eye blink.

### [Supplementary Note 6]

The information processing apparatus according to any one of Supplementary Notes 3 to 5, wherein
the applying unit applies the stimulus by outputting toward the target person at least one of a picture and a sound that increases tension of the target person and at least one of a picture and a sound that relieves the tension of the target person, and
the correlation includes a correlation between the stimulus and at least one of a time of the eye blink, a speed of moving eyelids by the eye blink, and a frequency of the eye blink.

### [Supplementary Note 7]

The information processing apparatus according to any one of Supplementary Notes 3 to 6, wherein
the applying unit applies the stimulus by outputting a task that increases tension of the target person and by encouraging the target person to take a rest to relieve the tension of the target person, and
the correlation includes a correlation between the stimulus and at least one of a time of the eye blink, a speed of moving eyelids by the eye blink, and a frequency of the eye blink.

### [Supplementary Note 8]

The information processing apparatus according to Supplementary Notes 1 to 7, wherein
the vital reaction includes a reaction of adjusting focus of a crystalline lens of the target person, and
the correlation includes a correlation between the stimulus and an emission direction of reflected light from at least one of a front surface and a back surface of the crystalline lens on which light is incident from a point light source or from a picture that imitates the point light source.

### [Supplementary Note 9]

The information processing apparatus according to Supplementary Note 8, wherein the applying unit applies the stimulus by outputting to the target person a picture of at least one of an object that is gradually enlarged and an object that is gradually reduced.

### [Supplementary Note 10]

The information processing apparatus according to Supplementary Note 8 or 9, wherein the applying unit applies the stimulus by outputting to the target person a picture of an object that is enlarged or reduced and then becomes out of focus.

### [Supplementary Note 11]

The information processing apparatus according to any one of Supplementary Notes 1 to 10, wherein the vital reaction includes a movement of a face of the target person and a movement of a line of sight of the target person.

### [Supplementary Note 12]

The information processing apparatus according to Supplementary Note 11, wherein the applying unit applies the stimulus by irradiating the target person with light from a predetermined direction, outputting a picture from a predetermined direction to the target person, and/or outputting a sound from a predetermined direction to the target person.

### [Supplementary Note 13]

The information processing apparatus according to Supplementary Note 11 or 12, further including an authentication unit that authenticates the target person by using an image generated by an imaging apparatus imaging the target person, wherein
the predetermined direction is a direction in which the imaging apparatus exists when viewed from the target person.

### [Supplementary Note 14]

An information processing method including:
applying a stimulus including at least one of light, a picture, and a sound that induces a change in a vital reaction of a target person; and
determining whether or not the target person is a living body, on the basis of a correlation between the stimulus and the vital reaction that occurs in the target person to which the stimulus is applied.

### [Supplementary Note 15]

A recording medium on which a computer program that allows a computer to execute an information processing method is recorded,
the information processing method including:
applying a stimulus including at least one of light, a picture, and a sound that induces a change in a vital reaction of a target person; and
determining whether or not the target person is a living body, on the basis of a correlation between the stimulus and the vital reaction that occurs in the target person to which the stimulus is applied.

At least a part of the constituent components of each of the example embodiments described above can be combined with at least another part of the constituent components of each of the example embodiments described above, as appropriate. A part of the constituent components of each of the example embodiments described above may not be used. Furthermore, to the extent permitted by law, all the references (e.g., publications) cited in this disclosure are incorporated by reference as a part of the description of this disclosure.

This disclosure is allowed to be changed, if desired, without departing from the essence or spirit of this disclosure which can be read from the claims and the entire identification. An information processing apparatus, an information processing method, and a recording medium with such changes are also intended to be within the technical scope of this disclosure.

### Description of Reference Codes

1 Camera
2 Authentication apparatus
3 Spoofing determination apparatus
31 Arithmetic apparatus
311 Stimulation control unit
312 Spoofing determination unit
1000 Information processing apparatus
1001 Applying unit
1002 Determination unit
SYS Authentication apparatus
IMG person image

## Claims

1. An information processing apparatus comprising:
an applying unit that applies a stimulus including at least one of light, a picture, and a sound that induces a change in a vital reaction of a target person; and
a determination unit that determines whether or not the target person is a living body, on the basis of a correlation between the stimulus and the vital reaction that occurs in the target person to which the stimulus is applied.

2. The information processing apparatus according to claim 1, wherein
the applying unit applies the stimulus to a first part of the target person, and
the correlation includes a correlation between the stimulus and the vital reaction in a second part of the target person that is different from the first part to which the stimulus is applied.

3. The information processing apparatus according to claim 1 or 2, wherein the vital reaction includes an eye blink.

4. The information processing apparatus according to claim 3, wherein
the applying unit applies the stimulus by outputting a picture of a living body blinking, toward the target person, and
the correlation includes a correlation between the stimulus and at least one of a time of the eye blink and a frequency of the eye blink.

5. The information processing apparatus according to claim 3 or 4, wherein
the applying unit applies the stimulus by outputting toward the target person at least one of a picture and a sound that increases tension of the target person, and
the correlation includes a correlation between the stimulus and at least one of a time of the eye blink, a speed of moving eyelids by the eye blink, and a frequency of the eye blink.

6. The information processing apparatus according to any one of claims 3 to 5, wherein
the applying unit applies the stimulus by outputting toward the target person at least one of a picture and a sound that increases tension of the target person and at least one of a picture and a sound that relieves the tension of the target person, and
the correlation includes a correlation between the stimulus and at least one of a time of the eye blink, a speed of moving eyelids by the eye blink, and a frequency of the eye blink.

7. The information processing apparatus according to any one of claims 3 to 6, wherein
the applying unit applies the stimulus by outputting a task that increases tension of the target person and by encouraging the target person to take a rest to relieve the tension of the target person, and
the correlation includes a correlation between the stimulus and at least one of a time of the eye blink, a speed of moving eyelids by the eye blink, and a frequency of the eye blink.

8. The information processing apparatus according to claims 1 to 7, wherein
the vital reaction includes a reaction of adjusting focus of a crystalline lens of the target person, and
the correlation includes a correlation between the stimulus and an emission direction of reflected light from at least one of a front surface and a back surface of the crystalline lens on which light is incident from a point light source or from a picture that imitates the point light source.

9. The information processing apparatus according to claim 8, wherein the applying unit applies the stimulus by outputting to the target person a picture of at least one of an object that is gradually enlarged and an object that is gradually reduced.

10. The information processing apparatus according to claim 8 or 9, wherein the applying unit applies the stimulus by outputting to the target person a picture of an object that is enlarged or reduced and then becomes out of focus.

11. The information processing apparatus according to any one of claims 1 to 10, wherein the vital reaction includes a movement of a face of the target person and a movement of a line of sight of the target person.

12. The information processing apparatus according to claim 11, wherein the applying unit applies the stimulus by irradiating the target person with light from a predetermined direction, outputting a picture from a predetermined direction to the target person, and/or outputting a sound from a predetermined direction to the target person.

13. The information processing apparatus according to claim 11 or 12, further comprising an authentication unit that authenticates the target person by using an image generated by an imaging apparatus imaging the target person, wherein
the predetermined direction is a direction in which the imaging apparatus exists when viewed from the target person.

14. An information processing method comprising:
applying a stimulus including at least one of light, a picture, and a sound that induces a change in a vital reaction of a target person; and
determining whether or not the target person is a living body, on the basis of a correlation between the stimulus and the vital reaction that occurs in the target person to which the stimulus is applied.

15. A recording medium on which a computer program that allows a computer to execute an information processing method is recorded,
the information processing method including:
applying a stimulus including at least one of light, a picture, and a sound that induces a change in a vital reaction of a target person; and
determining whether or not the target person is a living body, on the basis of a correlation between the stimulus and the vital reaction that occurs in the target person to which the stimulus is applied.
